# EUROPÄISCHE PATENTSCHRIFT

(11) **EP 1 558 159 B1**
(45) Veröffentlichungstag und Bekanntmachung des Hinweises auf die Patenterteilung: **14.11.2007**
(21) Anmeldenummer: 02808064.6
(22) Anmeldetag: 29.10.2002
(51) Int. Cl.: A61B 17/74

(54) **VORRICHTUNG ZUR BEHANDLUNG VON FRAKTUREN DES FEMUR**
DEVICE FOR THE TREATMENT OF FRACTURES OF THE FEMUR
DISPOSITIF PERMETTANT DE TRAITER DES FRACTURES DU FEMUR

(43) Veröffentlichungstag der Anmeldung: 03.08.2005
(73) Patentinhaber: Synthes GmbH, 4436 Oberdorf (CH)
(72) Erfinder: FRIGG, Robert, CH-2544 Bettlach (CH); HATTLER, Eric, CH-4500 Solothurn (CH); WIDMER, Walter, CH-4515 Oberdorf (CH); BARRIOS, Elena, CH-2562 Port (BE) (CH)
(74) Vertreter: Lusuardi, Werther
(86) Internationale Anmeldenummer: PCT/CH2002/000584
(87) Internationale Veröffentlichungsnummer: WO 2004/039270

(56) Entgegenhaltungen:
- DE-A- 19 723 339
- DE-B- 1 071 285
- US-A- 5 324 292
- US-A- 5 454 813

## Beschreibung

Die Erfindung betrifft eine Vorrichtung zur Behandlung von Frakturen des Femur gemäss dem Oberbegriff des Patentanspruchs 1 sowie einen intramedullären Marknagel zur Verwendung in einer solchen Vorrichtung gemäss dem Oberbegriff des Anspruchs 18.

Es sind bereits Vorrichtungen bekannt, bei welchen eine Rotationssicherung des Femurkopfes mit einer einzigen Hüftschraube, d.h. einem longitudinalen Knochenfixationsmittel zu erreichen versucht wird. Aus der EP-B 0 441 577 ist beispielsweise eine solche Vorrichtung bekannt, welche eine die Hüftschraube gleitend aufnehmende Hülse aufweist, wobei die Hülse mittels einer von proximal einführbaren Blockierschraube im Marknagel gegen Rotation gesichert werden kann. Der Schaft der Hüftschraube und die Bohrung der Hülse sind allerdings unrund ausgebildet, damit sich die Hüftschraube in der Hülse nicht drehen kann. Die Hüftschraube muss sich aber während des Einbringens in den Hüftkopf drehen können. Aus diesem Grund muss bei der Implantation zuerst die Hüftschraube eingesetzt werden und erst danach die Gleithülse. Die beiden Elemente können nicht zusammen eingeführt werden, so dass die Operation kompliziert wird. Im weiteren besteht die Gefahr, dass die Hüftschraube nach medial wandert, wenn nicht zusätzlich eine Kompressionsschraube verwendet wird. Ein weiterer Nachteil besteht darin, dass die Blockierschraube von oben (kranial) in den Marknagel eingeführt werden muss, was einen zusätzlichen Operationsschritt bedeutet. Schliesslich ist bei einer allfällig später notwendig werdenden Entfernung der Hüftschraube ein relativ grosser Eingriff notwendig, um in einem Schritt die von proximal in den Marknagel eingeschraubte Blockierschraube zu lösen, bevor die Hüftschraube entfernt werden kann.

Im weiteren ist aus der US-A 5,454,813 LAWES ein Marknagel mit einer Hüftschraube und mit einer Gleithülse bekannt, bei welchem der Durchgang im Marknagel, das äussere und innere Profil der Gleithülse und der Schaft der Hüftschraube unrund ausgebildet sind. Die Gleithülse wirkt hier somit als Antirotationsmittel zwischen der Hüftschraube und dem Marknagel. Auch diese bekannte Vorrichtung weist die gleichen Nachteile auf wie die EP-B 0 441 577, d.h. eine komplizierte Operationstechnik, sowohl beim Implantieren wie auch beim Explantieren der Hüftschraube und auch die mediale Wanderung kann nur mit einem zusätzlichen Bauelement (einem Spannungseinsteller) verhindert werden.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung auch tatsächlich publiziert oder öffentlich bekannt war.

Hier will die Erfindung Abhilfe schaffen. Der Erfindung liegt das Problem zugrunde, eine Vorrichtung zur Behandlung von Frakturen des Femur, insbesondere von proximalen Femurfrakturen zu schaffen, welche eine einfache und sichere Rotationsblockierung zwischen dem longitudinalen Knochenfixationselement (z.B. einer Hüftschraube) und dem Marknagel erlaubt, die Operationsmethode vereinfacht und verkürzt und das laterale Gleiten des longitudinalen Knochenfixationselementes nicht limitiert.

Die Erfindung löst die gestellte Aufgabe mit einer Vorrichtung, welche die Merkmale des Anspruchs 1, sowie einem Marknagel, welcher die Merkmale des Anspruchs 18 aufweist.

Mit der erfindungsgemässen Vorrichtung ist der Vorteil erzielbar, dass sich der vorzugsweise als Schraube oder Helixklinge ausgebildete Vorderteil des longitudinalen Knochenfixationselementes optimal in der Spongiosa des Femurkopfes verankern kann, weil der Schaft des longitudinalen Knochenfixationselement in der ihn umschliessenden Gleithülse rotativ frei beweglich bleibt und die Gleithülse mit dem darin befindlichen longitudinalen Knochenfixationselement gleichzeitig axial frei beweglich bleibt.

Damit die klinische Handhabung dieser Kombination aus longitudinalem Knochenfixationselement + Gleithülse einfach bleibt, können die beiden Elemente vorzugsweise so vormontiert werden, dass sich das longitudinale Knochenfixationselement zwar frei in der Gleithülse drehen kann aber gegenüber dieser axial fixiert ist. Diese so vormontierte Konstruktion hat den Vorteil, dass das longitudinalen Knochenfixationselement beim Einbringen in den Femurkopf nicht speziell ausgerichtet werden muss und sich beim Einschlagen helixförmig in den Femurkopf drehen kann. Das longitudinale Knochenfixationselement ist in diesem Zeitpunkt noch nicht auf Rotation gesichert. Der Chirurg kann somit noch Rotationskorrekturen des Femurkopfes vornehmen, bevor er die Rotation des longitudinalen Knochenfixationselementes, durch das Eindrehen der Fixationsschraube von lateral her, in die Bohrung des Schaftes des longitudinalen Knochenfixationselementes blockiert.

Eine bevorzugte Weiterbildung besteht darin, dass am freien Ende des Schaftes des longitudinalen Knochenfixationselementes eine koaxial zur Längsachse angeordnete Bohrung vorgesehen ist, welche vorzugsweise mit einem Innengewinde versehen ist.

Bei einer speziellen Ausführungsform bestehen die Blockiermittel aus einer Feststellschraube mit einem Schraubenkopf des Durchmessers D und einem ein Aussengewinde tragenden Schraubenschaft des Durchmessers d, wobei D > d ist. Dabei korrespondiert das Aussengewinde des Schraubenschaftes mit dem Innengewinde der Bohrung des Schaftes des longitudinalen Knochenfixationselementes und ist in dessen Bohrung soweit eindrehbar, bis der Schraubenkopf auf das hintere Ende der Gleithülse auftrifft und durch weiteres Festziehen der als Blockiermittel wirkenden Feststellschraube eine kraftschlüssige Verbindung zwischen dem longitudinalen Knochenfixationselement und der Gleithülse resultiert.

Bei einer weiteren Ausführungsform ist der Schaft des longitudinalen Knochenfixationselementes rotativ aber axial fest in der Gleithülse gelagert. Zu diesem Zweck kann der Schaft des longitudinalen Knochenfixationselementes mit einer ersten Ringnut und die innere Mantelfläche der Gleithülse mit einer zweiten Ringnut versehen sein. Im weiteren ist ein die axiale Beweglichkeit des Schaftes in der Gleithülse blockierendes Element, vorzugsweise in Form eines in die beiden Ringnuten eingreifenden Ringes vorgesehen. Statt des Ringes könnten aber auch andere blockierende Elemente (z.B. in Form eines Stiftes) verwendet werden.

Bei einer weiteren Ausführungsform ragt das hintere Ende der Gleithülse um einen definierten Betrag x über das freie Ende des Schaftes des longitudinalen Knochenfixationselementes hervor, vorzugsweise um mindestens 0,01 mm.

Bei einer anderen speziellen Ausführungsform ist am freien Ende des Schaftes - statt des Innengewindes - ein Aussengewinde vorgesehen. Das Blockiermittel besteht bei dieser Ausführungsform - statt der Feststellschraube - aus einer Mutter mit einem zum Aussengewinde des Schaftes korrespondierenden Innengewinde.

Der unrunde Querschnitt des Durchgangs des intramedullären Nagels kann bei einer speziellen Ausführungsform periphere Teilabschnitte in Form von Teilkreisbögen aufweisen. Diese Kombination eines unrunden mit einem runden Querschnitt erlaubt die Verwendung dieses derart ausgestalteten Marknagels einerseits mit der erfindungsgemässen unrunden Gleithülse anderseits aber auch ohne Gleithülse, lediglich mit einem longitudinalen Knochenfixationselement (z.B. einer Hüftschraube), welches einen runden Schaft aufweist, bei dem dank dieser Gestaltung trotz des unrunden Durchgangs eine gute Führung des runden Schaftes des Knochenfixationselementes (z.B. einer Hüftschraube) resultiert. Der solcherart ausgebildete Marknagel kann somit, sofern er eine zweite Querbohrung aufweist weiterhin auch in konventioneller Weise mit zwei Knochenfixationselementen (z.B. zwei Hüftschrauben verwendet werden.

Die Fixationsmittel des longitudinalen Knochenfixationselementes bestehen bevorzugt aus einer Helixklinge, vorzugsweise einer Doppelhelixklinge. Die Fixationsmittel können aber auch aus einem Schraubengewinde mit relativ geringer Steigung, einem Meissel, einem Nagel, einem T-Profil oder einem Doppel-T-Profil bestehen.

Bei einer besonderen Ausführungsform ist der Kopfteil des longitudinalen Knochenfixationselementes als mehrgängiges Gewinde, vorzugsweise als viergängiges Gewinde ausgebildet. Durch diese Ausgestaltung spielt die Positionierung des Knochenfixationselementes keine Rolle mehr wie beim eingängigen Gewinde. Das Gewinde des Kopfteils kann dabei eine Steigung von mindestens 50 mm, vorzugsweise von mindestens 80 mm aufweisen. Der Vorteil dieser relativ hohen Steigung liegt im höheren Widerstand gegen Rotation des Knochenfixationselementes. Zudem zerstört das als Helixklinge ausgebildete Knochenfixationselement weniger Knochensubstanz als eine konventionelle Hüftschraube mit relativ geringer Gewindesteigung. Der Knochen wird durch die Helixflächen der Helixklinge mehr kompaktiert als geschnitten.

Die Blockiermittel, welche in Form einer Feststellschraube oder einer Mutter realisiert sein können, sind vorzugsweise derart dimensioniert sind, dass sie bezüglich des Durchgangs des intramedullären Nagels als axialer Stop wirken. Dieser Stop vermeidet eine zu weite Migration des Knochenfixationsmittels nach medial.

Die Erfindung und Weiterbildungen der Erfindung werden im folgenden anhand der teilweise schematischen Darstellung mehrerer Ausführungsbeispiele noch näher erläutert.

Es zeigen:
Fig. 1 eine Seitenansicht durch die erfindungsgemässe Vorrichtung;
Fig. 2 einen partiellen Schnitt durch einen Marknagel im Bereich seines schrägen Durchgangs mit einem eingesetzten longitudinalen Knochenfixationselement und einer Gleithülse;
Fig. 3 einen partiellen Schnitt durch einen modifizierten Marknagel im Bereich seines schrägen Durchgangs mit einem eingesetzten longitudinalen Knochenfixationselement und einer Gleithülse;
Fig. 4 einen partiellen Schnitt durch eine weitere Modifikation eines Marknagels im Bereich seines schrägen Durchgangs mit einem eingesetzten longitudinalen Knochenfixationselement und einer Gleithülse;
Fig. 5 einen partiellen Längsschnitt durch die Vorrichtung nach Fig. 1;
Fig. 6 einen vergrösserten Ausschnitt des Kreises VI in Fig. 5;
Fig. 7 einen Schnitt längs der Linie VII-VII in Fig. 5;
Fig. 8 einen Längsschnitt durch eine modifizierte Gleithülse mit darin vormontierter Helix-Schraube; und
Fig. 9 einen Längsschnitt durch eine Modifikation der Gleithülse nach Fig. 8 mit einer darin vormontierten Helix-Schraube.

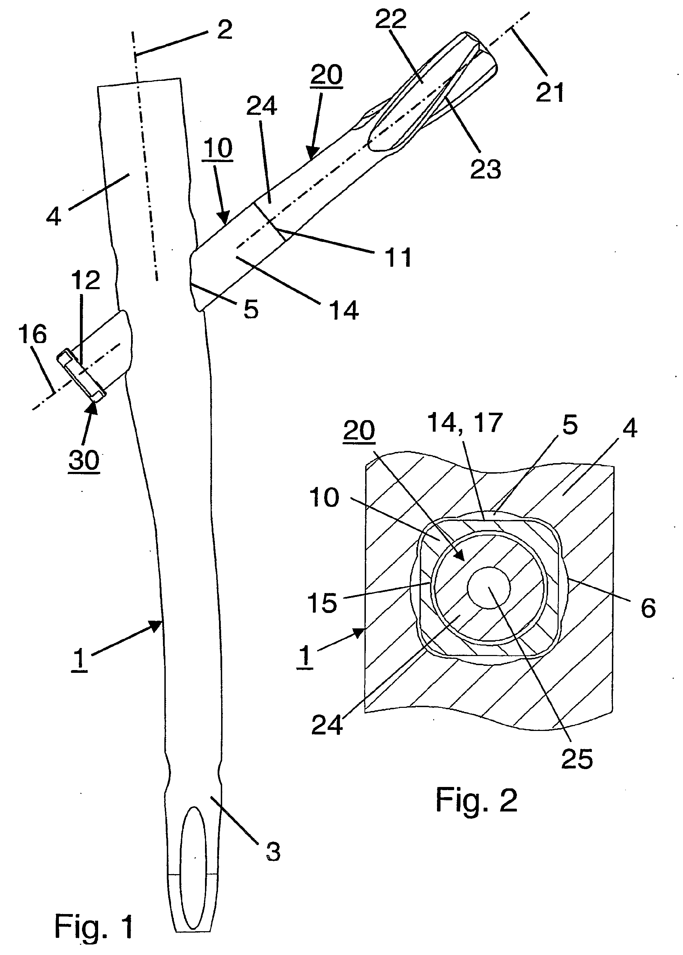

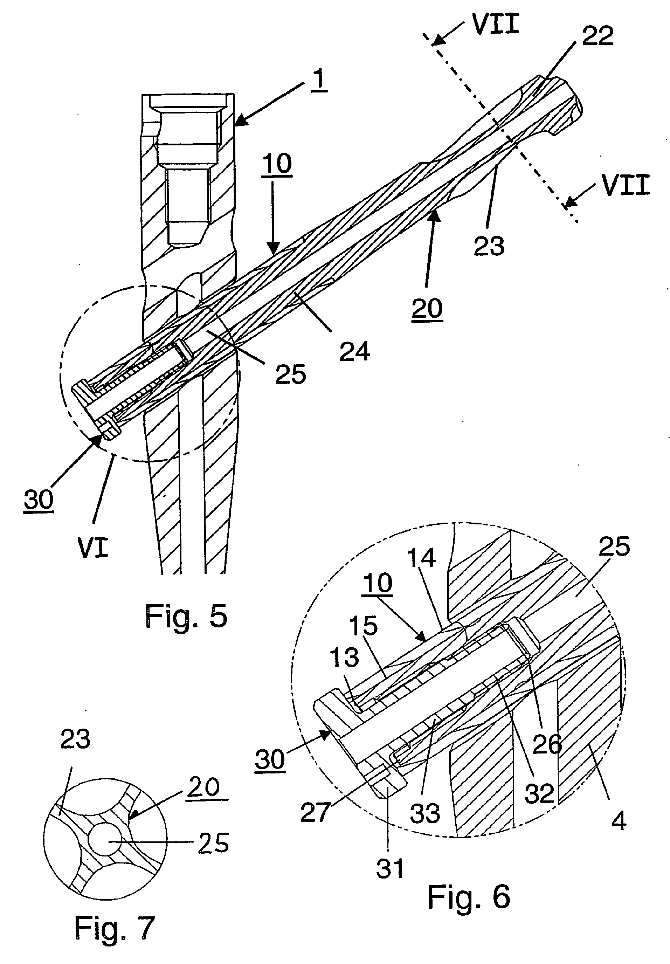

In den Fig. 1 - 2 sowie 5 - 7 ist eine Vorrichtung zur Behandlung von Frakturen des Femur dargestellt, welche einen intramedullären Nagel 1, eine Gleithülse 10, ein longitudinales Knochenfixationselement 20 in Form einer Helixschraube und ein Blockiermittel 30 in Form einer Feststellschraube umfasst.
Der intramedullären Nagel 1 besitzt eine zentrale Längsachse 2, einen in den Markkanal des Femur einführbaren Vorderteil 3, einen Hinterteil 4, sowie einen den Hinterteil 4, schräg zur Längsachse 2 durchbohrenden Durchgang 5 mit einem unrunden Querschnitt 6.
Die durch den unrunden Durchgang 5 hindurchführbare Gleithülse 10 besitzt ein vorderes Ende 11, ein hinteres Ende 12, eine zentrale Längsbohrung 13, eine äussere Mantelfläche 14, eine innere Mantelfläche 15 sowie eine Längsachse 16.
Das longitudinale Knochenfixationselement 20 in Form einer Helixschraube besitzt eine Längsachse 21, einen Kopfteil 22 mit Fixationsmitteln 23 in Form eines mehrgängigen Gewindes relativ hoher Steigung, die bei Benutzung mit dem Femurkopf in Eingriff bringbar sind, sowie einem, koaxial in die Gleithülse 10 einführbaren Schaft 24.
Die äussere Mantelfläche 14 der Gleithülse 10 weist einen unrunden Querschnitt 17 auf, während die innere Mantelfläche 15 der Gleithülse 10 einen runden Querschnitt 18 aufweist. Schliesslich sind Blockiermittel 30 in Form einer Feststellschraube vorgesehen, um die Rotation des longitudinalen Knochenfixationselementes 20 in der Gleithülse 10 wahlweise blockieren zu können. Am freien Ende 27 des Schaftes 24 des longitudinalen Knochenfixationselementes 20 ist eine koaxial zur Längsachse 21 angeordnete Bohrung 25 vorgesehen, welche mit einem Innengewinde 26 versehen ist.
Die Blockiermittel 30 in Form einer Feststellschraube weisen einen Schraubenkopf 31 des Durchmessers D und einen ein Aussengewinde 32 tragenden Schraubenschaft 33 des Durchmessers d auf, wobei D > d ist (Fig. 6). Das Aussengewinde 32 des Schraubenschaftes 33 korrespondiert mit dem Innengewinde 26 der Bohrung 25 des Schaftes 24 des longitudinalen Knochenfixationselementes 20 und ist in der Bohrung 25 soweit eindrehbar, bis der Schraubenkopf 31 auf das hintere Ende 12 der Gleithülse 10 auftrifft und durch ein weiteres Festziehen der als Blockiermittel 30 wirkenden Feststellschraube eine kraftschlüssige Verbindung zwischen dem longitudinalen Knochenfixationselement 20 und der Gleithülse 10 resultiert.

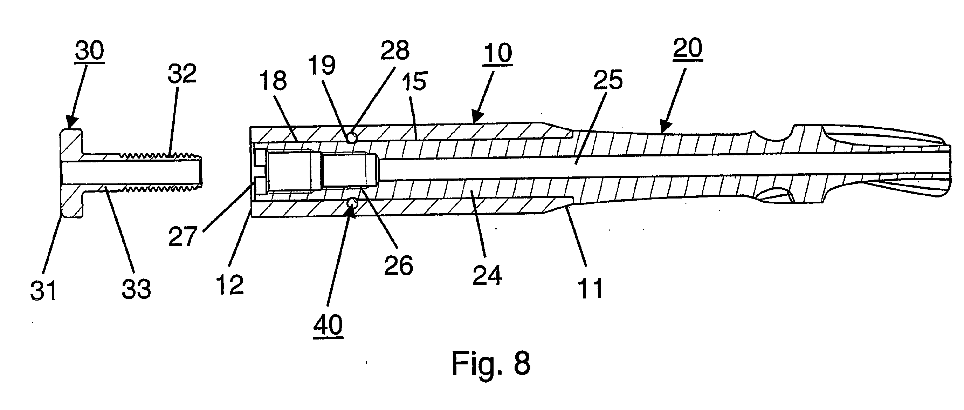

Bei einer weiteren in Fig. 8 dargestellten Ausführungsform ist der Schaft 24 des longitudinalen Knochenfixations-elementes 20 mit einer ersten Ringnut 28 und die innere Mantelfläche 15 der Gleithülse 10 mit einer zweiten Ringnut 19 versehen. In diese beiden Ringnuten 28,19 ist ein die axiale Beweglichkeit des Schaftes 24 in der Gleithülse 10 blockierendes Element 40 in Form eines Ringes gelagert. Dadurch ist der Schaft 24 des longitudinalen Knochenfixationselementes 20 rotativ aber axial fest in der Gleithülse 10 gelagert. Das hintere Ende 12 der Gleithülse 10 ragt dabei um einen definierten Betrag x über das freie Ende 27 des Schaftes 24 des longitudinalen Knochenfixationselementes 20 hervor, um einen sicheren Kraftschluss beim Anziehen der Feststellschraube zu garantieren. Durch diesen Kraftschluss ist es möglich die Rotation des longitudinalen Knochenfixationselementes 20 in der Gleithülse 10 wahlweise zu blockieren.

In Fig. 2 ist dargestellt, wie der kreiszylindrische Schaft 24 des als Helixklinge ausgebildeten, longitudinalen Knochenfixationselementes 20 im Inneren der ebenfalls eine kreiszylindrische innere Mantelfläche 15 aufweisenden Gleithülse 10 rotativ frei gelagert ist. Die im wesentlichen einen quadratischen, d.h. unrunden Querschnitt 17 aufweisende äussere Mantelfläche 14 der Gleithülse 10 ist dagegen im ebenfalls einen unrunden Querschnitt 6 aufweisenden Durchgang 5 des Marknagels 1 gegen Rotation gesichert. Der unrunde Querschnitt 6 des Durchgangs 5 ist - wie in Fig. 2 dargestellt - ebenfalls annähernd quadratisch ausgebildet, weist aber zusätzlich periphere Teilabschnitte in Form von Teilkreisbögen auf. Dank dieser Gestaltung kann der Marknagel 1 auch mit einer konventionellen Hüftschraube mit kreiszylindrischen Schaft verwendet werden, d.h. auch ohne Gleithülse.

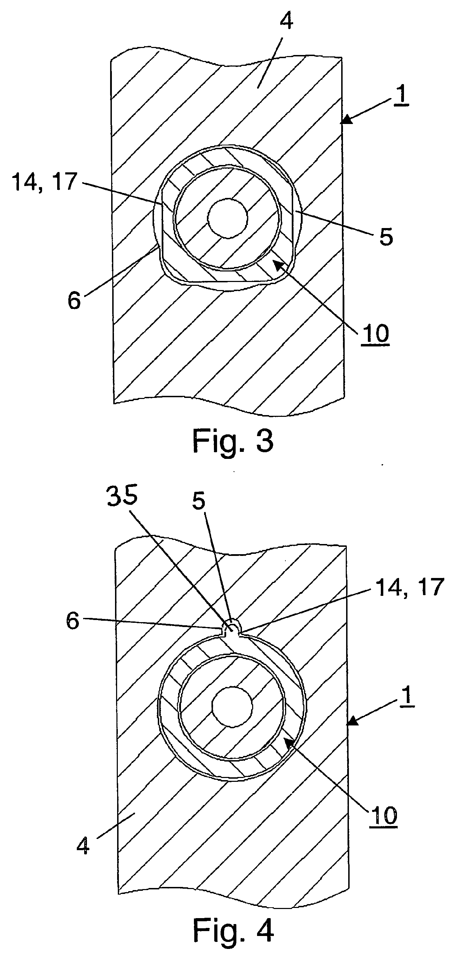

In Fig. 3 ist eine Variante eines unrunden Querschnitts 6 des Durchgangs 5 dargestellt, bei welchem zwei kleine Teilkreisbögen und ein grösserer Teilkreisbogen vorhanden sind. Entsprechend ist der unrunde Querschnitt 17 der äusseren Mantelfläche 14 der Gleithülse 10 ausgebildet. Gegenüber der Ausführung nach Fig. 2 ergibt sich ein einfacheres Handling, da wegen der bestehenden Symmetrie ein falsches Einsetzen der Hüftschraube ausgeschlossen ist.

In Fig. 4 ist noch eine weitere Variante eines unrunden Querschnitts 6 des Durchgangs 5 dargestellt, bei welchem der Querschnitt im wesentlichen zwar rund ist, aber eine Längsnut besitzt in welcher ein Längssteg 35 an der äusseren Mantelfläche 14 der Gleithülse 10 formschlüssig gelagert ist, so das die Rotation der beiden Elemente gesichert ist.

Wie in Fig, 7 dargestellt, sind die fixationsmittel 23 des longitudinalen Knochenfixationselementes nach Fig. 5 als viergängige Helix-Schraube ausgebildet, wobei das Gewinde eine Steigung von ca. 120 mm aufweist.

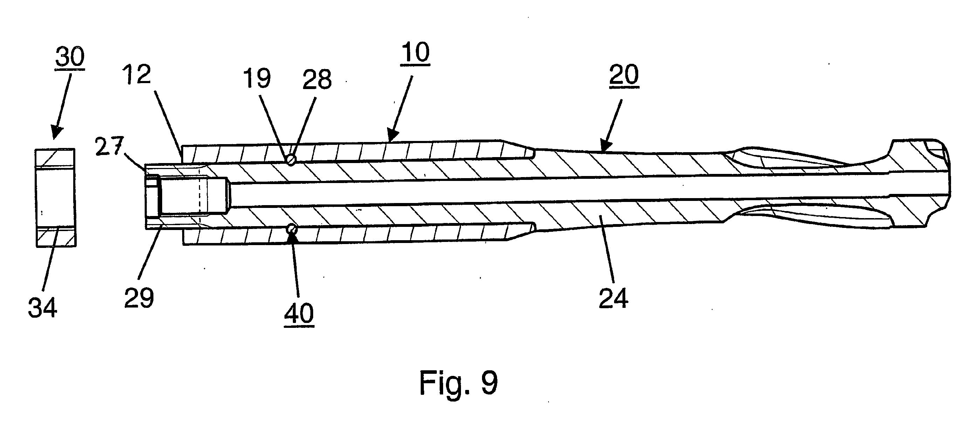

In Fig. 9 ist eine Variante der erfindungsgemässen Vorrichtung dargestellt bei welcher statt einer als Blockiermittel 30 wirkenden Feststellschraube eine Mutter vorgesehen ist. Dementsprechend weist das freie Ende 27 des Schaftes 24 des longitudinalen Knochenfixationselementes 20 statt eines Innengewindes ein Aussengewinde 29 auf, welches zum Innengewinde 34 der Mutter (Blockiermittel 30) korrespondiert. Innengewinde 34 und Aussengewinde 29 sind kreiszylindrisch ausgebildet. Das freie Ende 27 des Schaftes 24 des longitudinalen Knochenfixationselementes 20 ragt dabei um einen definierten Betrag x über das hintere Ende 12 der Gleithülse 10 hervor, damit die Mutter auf das Aussengewinde 29 aufgeschraubt werden kann. Sobald die Mutter auf das hintere Ende 12 der Gleithülse 10 stösst, entsteht ein sicherer Kraftschluss beim weiteren Verdrehen der Mutter. Durch diesen Kraftschluss ist es möglich die Rotation des longitudinalen Knochenfixationselementes 20 in der Gleithülse 10 wahlweise zu blockieren.

## Patentansprüche

1. Vorrichtung zur Behandlung von Frakturen des Femur mit
A) einem intramedullären Nagel (1) mit einer zentralen Längsachse (2), einem in den Markkanal des Femur einführbaren Vorderteil (3), einem Hinterteil (4), einen den Hinterteil (4), schräg zur Längsachse (2) durchbohrenden Durchgang (5) mit unrundem Querschnitt (6);
B) einer durch den unrunden Durchgang (5) hindurchführbaren Gleithülse (10), mit einem vorderen Ende (11), einem hinteren Ende (12), einer zentralen Längsbohrung (13), einer äusseren Mantelfläche (14), einer inneren Mantelfläche (15) und einer Längsachse (16);
C) einem longitudinalen Knochenfixationselement (20) mit einer Längsachse (21), einem Kopfteil (22) mit Fixationsmitteln (23), die bei Benutzung mit dem Femurkopf in Eingriff bringbar sind, sowie einem, koaxial in die Gleithülse (10) einführbaren Schaft (24); wobei
D) die äussere Mantelfläche (14) der Gleithülse (10) mindestens in einem Teilbereich einen unrunden Querschnitt (17) aufweist;
**dadurch gekennzeichnet, dass**
E) die innere Mantelfläche (15) der Gleithülse (10) einen runden Querschnitt (18) aufweist; und
F) Blockiermittel (30) vorgesehen sind, um die Rotation des longitudinalen Knochenfixationselementes (20) in der Gleithülse (10) wahlweise zu blockieren.

2. Vorrichtung nach Anspruch 1, **dadurch gekennzeichnet, dass** am freien Ende (27) des Schaftes (24) eine koaxial zur Längsachse (21) angeordnete Bohrung (25) vorgesehen ist, welche vorzugsweise mit einem Innengewinde (26) versehen ist.

3. Vorrichtung nach Anspruch 1 oder 2, **dadurch gekennzeichnet, dass** die Blockiermittel (30) eine Feststellschraube mit einem Schraubenkopf (31) des Durchmessers D und einem ein Aussengewinde (32) tragenden Schraubenschaft (33) des Durchmessers d vorgesehen ist, wobei D > d ist.

4. Vorrichtung nach Anspruch 3, **dadurch gekennzeichnet, dass** das Aussengewinde (32) des Schraubenschaftes (33) mit dem Innengewinde (26) der Bohrung (25) des Schaftes (24) des longitudinalen Knochenfixationselementes (20) korrespondiert und in der Bohrung (25) soweit eindrehbar ist, bis der Schraubenkopf (31) auf das hintere Ende (12) der Gleithülse (10) auftrifft und durch weiteres Festziehen der als Blockiermittel (30) wirkenden Feststellschraube eine kraftschlüssige Verbindung zwischen dem longitudinalen Knochenfixationselement (20) und der Gleithülse (10) resultiert.

5. Vorrichtung nach einem der Ansprüche 1 bis 4, **dadurch gekennzeichnet, dass** der Schaft (24) des longitudinalen Knochenfixationselementes (20) rotativ aber axial fest in der Gleithülse (10) gelagert ist.

6. Vorrichtung nach Anspruch 5, **dadurch gekennzeichnet, dass** der Schaft (24) des longitudinalen Knochenfixationselementes (20) mit einer ersten Ringnut (28) und die innere Mantelfläche (15) der Gleithülse (10) mit einer zweiten Ringnut (19) versehen sind und dass ein die axiale Beweglichkeit des Schaftes (24) in der Gleithülse (10) blockierendes Element (40), vorzugsweise in Form eines in die beiden Ringnuten (28,19) eingreifenden Ringes vorgesehen ist.

7. Vorrichtung nach Anspruch 6, **dadurch gekennzeichnet, dass** das hintere Ende (12) der Gleithülse (10) um einen definierten Betrag x über das freie Ende (27) des Schaftes (24) des longitudinalen Knochenfixationselementes (20) hervorragt, vorzugsweise um mindestens x > 0,01 mm.

8. Vorrichtung nach Anspruch 1, **dadurch gekennzeichnet, dass** am freien Ende (27) des Schaftes (24) ein Aussengewinde (29) vorgesehen ist.

9. Vorrichtung nach Anspruch 8, **dadurch gekennzeichnet, dass** das Blockiermittel (30) eine Mutter ist mit einem zum Aussengewinde (29) des Schaftes (24) korrespondierenden Innengewinde (34).

10. Vorrichtung nach einem der Ansprüche 1 bis 9, **dadurch gekennzeichnet, dass** der unrunde Querschnitt (6) des Durchgangs (5) periphere Teilabschnitte in Form von Teilkreisbögen aufweist.

11. Vorrichtung nach einem der Ansprüche 1 bis 10, **dadurch gekennzeichnet, dass** die Fixationsmittel (23) des longitudinalen Knochenfixationselementes (20) eine Helixklinge, vorzugsweise eine Doppelhelixklinge sind.

12. Vorrichtung nach einem der Ansprüche 1 bis 11, **dadurch gekennzeichnet, dass** die Fixationsmittel (23) ein Schraubengewinde, ein Meissel, ein Nagel, ein T-Profil oder ein Doppel-T-Profil sind.

13. Vorrichtung nach einem der Ansprüche 1 bis 12, **dadurch gekennzeichnet, dass** der Kopfteil (22) des longitudinalen Knochenfixationselementes (20) als mehrgängiges Gewinde, vorzugsweise als viergängiges Gewinde ausgebildet ist.

14. Vorrichtung nach einem der Ansprüche 1 bis 13, **dadurch gekennzeichnet, dass** das Gewinde des Kopfteils (22) eine Steigung von mindestens 50 mm, vorzugsweise mindestens 80 mm aufweist.

15. Vorrichtung nach einem der Ansprüche 1 bis 14, **dadurch gekennzeichnet, dass** die Blockiermittel (30), welche vorzugsweise in Form einer Feststellschraube oder eine Mutter realisiert sind, derart dimensioniert sind, dass sie bezüglich des Durchgangs (5) als axialer Stop wirken.

16. Vorrichtung nach einem der Ansprüche 1 bis 15, **dadurch gekennzeichnet, dass** das longitudinale Knochenfixationselement (20) eine Hüftschraube ist.

17. Vorrichtung nach einem der Ansprüche 1 bis 15, **dadurch gekennzeichnet, dass** das longitudinale Knochenfixationselement (20) eine Helix-Schraube ist.

## Claims

1. A device for the treatment of femoral fractions with
A) an intramedullary pin (1) with a central axis (2), a front portion (3) that can be introduced into the medullary canal of the femur, a rear portion (4), a passage (5) with a non-circular cross-section (6) that passes through the rear portion (4) obliquely to the longitudinal axis (2),
B) a sliding sleeve (10), that can pass through the non-circular passage (5), with a front end (11), a rear end (12), a central longitudinal bore (13), an external jacket surface (14), an internal jacket surface (15) and a longitudinal axis (16),
C) a longitudinal bone fixing element (20) with a longitudinal axis (21), a head portion (22) with fixing means (23) that can engage the femoral head during use, as well as a shaft (24) that can be coaxially introduced into the sliding sleeve (10), wherein
D) the external jacket surface (14) of the sliding sleeve (10) has at least in part a non-circular cross-section (17),
**characterised in that**
E) the internal jacket surface (15) of the sliding sleeve (10) has a round cross-section (38), and
F) locking means (30) are provided to optionally block the rotation of the longitudinal bone fixing element (20) in the sliding sleeve (10).

2. A device according to claim 1, **characterised in that** at the free end (27) of the shaft (24) a bore (25) is provided coaxially with the longitudinal axis (21), that preferably has an inside thread (26).

3. A device according to claim 1 or 2, **characterised in that** the locking means (30) is a fixing screw with a screw head (31) having a diameter of D and a screw shank (33) with a diameter of d having an outside thread (32), while D > d.

4. A device according to claim 3, **characterised in that** the outside thread (32) of the screw shank (33) corresponds to the inside thread (26) of the bore (25) of the shaft (24) of the longitudinal bone fixing element (20) and can be screwed into the bore (25) until the screw head (31) abuts against the rear end (12) of the sliding sleeve (10) and a further tightening of the fixing screw, acting as locking means (30), will result in a force-locked connection between the longitudinal bone fixing element (20) and the sliding sleeve (10).

5. A device according to any one of claims 1 to 4, **characterised in that** the shaft (24) of the longitudinal bone fixing element (20) is so mounted in the sliding sleeve (10) that it can rotate but is axially fixed.

6. A device according to claim 5, **characterised in that** the shaft (24) of the longitudinal bone fixing element (20) has a first annular groove (28) and the internal jacket surface (15) of the sliding sleeve (10) a second annular groove (19) and that an element (40), blocking the axial displacement of the shaft (24) in the sliding sleeve (10), is provided preferably in the form of a ring, engaging the two annular grooves (28, 19).

7. A device according to claim 6, **characterised in that** the rear end (12) of the sliding sleeve (10) protrudes past the free end (27) of the shaft (24) of the longitudinal bone fixing element (20) by a specific amount x, preferably by at least 0,01 mm.

8. A device according to claim 1, **characterised in that** on the free end (27) of the shaft (24) an outside thread (29) is provided.

9. A device according to claim 8, **characterised in that** the blocking means (30) is a nut with an inside thread (34) that corresponds to the outside thread (29) of the shaft (24).

10. A device according to any one of claims 1 to 9, **characterised in that** the non-circular cross-section (6) of the passage (5) has peripheral part-sections in the form of partial circular arcs.

11. A device according to any one of claims 1 to 10, **characterised in that** the fixing means (23) of the longitudinal bone fixing element (20) is a helical blade, preferably a double helical blade.

12. A device according to any of claims 1 to 11, **characterised in that** the fixing means (23) is a screw thread, a chisel, a pin, a T-section or a double T-section.

13. A device according to any one of claims 1 to 12, **characterised in that** the head portion (22) of the longitudinal bone fixing element (20) is constructed as a multi-start thread, preferably as a four-start thread.

14. A device according to any one of claims 1 to 13, **characterised in that** the thread of the head portion (22) has a pitch of at least 50 mm, preferably at least 80 mm.

15. A device according to any one of claims 1 to 14, **characterised in that** the locking means (30), that are preferably realised in the form of a fixing screw or a nut, are so dimensioned that they act as an axial stop relative to the passage (5).

16. A device according to any one of claims 1 to 15, **characterised in that** the longitudinal bone fixing element (20) is a hip screw.

17. A device according to any of claims 1 to 15, **characterised in that** the longitudinal bone fixing element (20) is a helical screw.

## Revendications

1. Dispositif pour le traitement de fractures du fémur, comprenant
A) un clou intramédullaire (1) ayant un axe longitudinal central (2), une partie avant (3) pouvant être introduite dans le canal médullaire du fémur, une partie arrière (4), un passage (5) traversant la partie arrière (4) en biais par rapport à l'axe longitudinal (2), ayant un diamètre ovalisé (6) ;
B) une douille coulissante (10) pouvant être insérée dans le passage ovalisé (5), comprenant une extrémité avant (11), une extrémité arrière (12), un alésage longitudinal central (13), une enveloppe externe (14), une enveloppe interne (15) et un axe longitudinal (16) ;
C) un élément de fixation osseuse longitudinal (20) ayant un axe longitudinal (21), une partie de tête (22) comprenant des moyens de fixation (23) qui, en conditions d'utilisation, peuvent venir en prise avec la tête fémorale, et une tige (24) pouvant être introduite coaxialement dans la douille coulissante (10) ; dans lequel
D) l'enveloppe externe (14) de la douille coulissante (10) présente, au moins dans une zone partielle, un diamètre ovalisé (17) ;
**caractérisé en ce que**
E) l'enveloppe interne (15) de la douille coulissante (10) présente un diamètre rond (18) ; et
F) des moyens de blocage (30) sont prévus pour bloquer éventuellement la rotation de l'élément de fixation osseuse longitudinal (20) dans la douille coulissante (10).

2. Dispositif selon la revendication 1, **caractérisé en ce qu'**un alésage (25) coaxial à l'axe longitudinal (21), qui comprend de préférence un taraudage (26), est prévu au niveau de l'extrémité libre (27) de la tige (24).

3. Dispositif selon la revendication 1 ou 2, **caractérisé en ce que** les moyens de blocage (30) comprennent une vis de blocage présentant une tête de vis (31) de diamètre D et une tige de vis (33) de diamètre d comprenant un filetage (32), où D > d.

4. Dispositif selon la revendication 3, **caractérisé en ce que** le filetage (32) de la tige de vis (33) correspond au taraudage (26) de l'alésage (25) de la tige (24) de l'élément de fixation osseuse longitudinal (20), et qu'il peut être vissé dans l'alésage (25) jusqu'à ce que la tête de vis (31) atteigne l'extrémité arrière (12) de la douille coulissante (10) et qu'un serrage ultérieur de la vis de blocage servant de moyen de blocage (30) permette d'obtenir un assemblage par enfoncement forcé entre l'élément de fixation osseuse longitudinal (20) et la douille coulissante (10).

5. Dispositif selon l'une quelconque des revendications 1 à 4, **caractérisé en ce que** la tige (24) de l'élément de fixation osseuse longitudinal (20) est logée dans la douille coulissante (10) de manière à pouvoir tourner mais en restant fixe sur le plan axial.

6. Dispositif selon la revendication 5, **caractérisé en ce que** la tige (24) de l'élément de fixation osseuse longitudinal (20) comprend une première rainure annulaire (28) et l'enveloppe interne (15) de la douille coulissante (10) comprend une deuxième rainure annulaire (19), et **en ce qu'**un élément (40) bloquant le déplacement axial de la tige (24) dans la douille coulissante (10), de préférence sous la forme d'un anneau s'encliquetant dans les deux rainures annulaires (28, 19), est prévu.

7. Dispositif selon la revendication 6, **caractérisé en ce que** l'extrémité arrière (12) de la douille coulissante (10) dépasse de l'extrémité libre (27) de la tige (24) de l'élément de fixation osseuse longitudinal (20) sur une distance définie x, de préférence d'au moins x > 0,01 mm.

8. Dispositif selon la revendication 1, **caractérisé en ce qu'**un filetage (29) est prévu au niveau de l'extrémité libre (27) de la tige (24).

9. Dispositif selon la revendication 8, **caractérisé en ce que** le moyen de blocage (30) est un écrou avec un taraudage (34) correspondant au filetage (29) de la tige (24).

10. Dispositif selon l'une quelconque des revendications 1 à 9, **caractérisé en ce que** le diamètre ovalisé (6) du passage (5) présente des segments périphériques en forme d'arcs de cercles partiels.

11. Dispositif selon l'une quelconque des revendications 1 à 10, **caractérisé en ce que** les moyens de fixation (23) de l'élément de fixation osseuse longitudinal (20) sont une lame hélicoïdale, de préférence une lame bi-hélicoïdale.

12. Dispositif selon l'une quelconque des revendications 1 à 11, **caractérisé en ce que** les moyens de fixation (23) sont un filet de vis, un trépan, un clou, un profil en T ou un profil en double T.

13. Dispositif selon l'une quelconque des revendications 1 à 12, **caractérisé en ce que** la partie de tête (22) de l'élément de fixation osseuse longitudinal (20) est réalisée sous la forme d'un filetage multiple, de préférence d'un filetage quadruple.

14. Dispositif selon l'une quelconque des revendications 1 à 13, **caractérisé en ce que** le filetage de la partie de tête (22) présente un pas d'au moins 50 mm, de préférence d'au moins 80 mm.

15. Dispositif selon l'une quelconque des revendications 1 à 14, **caractérisé en ce que** les moyens de blocage (30), qui sont de préférence réalisés sous la forme d'une vis de blocage ou d'un écrou, sont dimensionnés de façon à servir de butée axiale par rapport au passage (5).

16. Dispositif selon l'une quelconque des revendications 1 à 15, **caractérisé en ce que** l'élément de fixation osseuse longitudinal (20) est une vis de hanche.

17. Dispositif selon l'une quelconque des revendications 1 à 15, **caractérisé en ce que** l'élément de fixation osseuse longitudinal (20) est une vis hélicoïdale.
